# EUROPEAN PATENT SPECIFICATION

(11) **EP 1 291 427 B1**
(45) Date of publication and mention of the grant of the patent: **13.12.2006**
(21) Application number: 01934418.3
(22) Date of filing: 30.05.2001
(51) Int. Cl.: C12N 15/53, C12N 9/02

(54) **METHOD OF DETECTING AND QUANTIFYING HUMAN P450 MOLECULAR SPECIES AND PROBE AND KIT FOR THIS METHOD**
VERFAHREN ZUR DETEKTION UND QUANTIFIZIERUNG VON MENSCHLICHEN P450-MOLEKÜLEN UND SONDE UND KIT FÜR DIESES VERFAHREN
TECHNIQUE DE DETECTION ET DE QUANTIFICATION DE L'ESPECE MOLECULAIRE HUMAINE P450, SONDE ET NECESSAIRE A CET EFFET

(30) Priority: 01.06.2000 JP 2000164214
(43) Date of publication of application: 12.03.2003
(60) Divisional application: 06014725.3
(73) Proprietor: OTSUKA PHARMACEUTICAL FACTORY, INC., Naruto-shi, Tokushima 772-8601 (JP)
(72) Inventor: NISHIMURA, Masuhiro, Tokushima-shi, Tokushima 779-3117 (JP); YAGUCHI, Hiroshi, Naruto-shi, Tokushima 772-0017 (JP); NAITO, Shinsaku, Tokushima-shi, Tokushima 771-0130 (JP); HIRAOKA, Isao, Tokushima-shi, Tokushima 770-0861 (JP)
(74) Representative: HOFFMANN EITLE
(86) International application number: PCT/JP2001/004544
(87) International publication number: WO 2001/092538

(56) References cited:
- EP-A2- 0 644 267
- WO-A-01/96555
- JP-A- 8 027 193
- JP-A- 8 027 194
- DATABASE EMBL [Online] 29 May 1995 (1995-05-29), HILLIER L. ET AL: "yh17f12.r1 Soares infant brain 1NIB Homo sapiens cDNA clone IMAGE: 37838 5' mRNA sequence" XP002274425 retrieved from EBI Database accession no. R59502.1
- C.A. HEID ET AL.: 'Real time quantitative PCR' GENOME RESEARCH vol. 6, no. 10, 1996, pages 986 - 994, XP002945498
- D.P. HARTLEY ET AL.: 'Detection of chemical-induced differential expression of rat hepatic cytochrome P450 MRNA transcripts using branched DNA signal amplification technology' DRUG METAB. DISPOS. vol. 28, no. 5, May 2000, pages 608 - 616, XP002945499
- JAISWAL A.K. ET AL.: 'Human dioxin-inducible cytochrome P1-450: complementary DNA and amino acid sequence' SCIENCE vol. 228, 05 April 1985, pages 80 - 83, XP002945500
- TANG Y.M. ET AL.: 'Isolation and characterization of the human cytochrome P450 CYP1B1 gene' J. BIOL. CHEM. vol. 271, no. 45, 08 November 1996, pages 28324 - 28330, XP002945901
- YAMANO S. ET AL.: 'The CYP2A3 gene product catlyzes coumarin 7-hydroxylation in human liver microsomes' BIOCHEMISTRY vol. 29, no. 5, 06 February 1990, pages 1322 - 1329, XP002945902
- OKINO S.T. ET AL.: 'Characterization of multiple human cytochrome P-450 1 cDNAs. The chromosomal localization of the gene and evidence for alternate RNA splicing' J. BIOL. CHEM. vol. 262, no. 33, 25 November 1987, pages 16072 - 16079, XP002945903
- ROMKES M. ET AL.: 'Cloning and expression of complementary DNAs for multiple members of the human cytochrome P450IIC subfamily' BIOCHEMISTRY vol. 30, no. 13, 02 April 1991, pages 3247 - 3255, XP002945904
- GONZALEZ F.J. ET AL.: 'Characterization of the common genetic defect inhumans deficient in debrisoquine metabolism' NATURE vol. 331, no. 6155, 04 February 1988, pages 442 - 446, XP002945905
- NHAMBURO P.T. ET AL.: 'The human CYP2F gene subfamily: indentification of a cDNA encoding a new cytochrome P450, cDNA-directed expression and chromosome mapping' BIOCHEMISTRY vol. 29, no. 23, 12 June 1990, pages 5491 - 5499, XP002945906
- WU S. ET AL.: 'Molecular cloning and expression of CYP2J2, a human cytochrome P450 arachidonic acid epoxygenase highly expressed in heart' J. BIOL. CHEM. vol. 271, no. 7, 16 February 1996, pages 3460 - 3468, XP002945907
- MOLOWA D.T. ET AL.: 'Complete cDNA sequence of a cytochrome P-450 inducible by glucocorticoids in human liver' PROC. NATL. ACAD. SCI. USA vol. 83, no. 14, July 1986, pages 5311 - 5315, XP002945908
- BYLUND J. ET AL.: 'Gene expression of a novel cytochrome P450 of the CYP4F subfamily in human seminal vesicles' BIOCHEM. BIOPHYS. RES. COMMUN. vol. 261, no. 1, 22 July 1999, pages 169 - 174, XP002945909
- AOYAMA T. ET AL.: 'Cytochrome P-450 hPCN3, a novel cytochrome P-450 IIIA gene product that is differentially expressed in adult human liver. cDNA and deduced amino acid sequence and distinct specificities of cDNA-expressed hPCN1 and hPCN3 for the metabolism of steroid hormones and cyclosporine' J. BIOL. CHEM. vol. 264, no. 18, 25 July 1989, pages 10388 - 10395, XP002945910
- MOLOWA D.T. ET AL.: 'Complete cDNA sequence of a cytochrome P-450 inducible by glucocorticoids in human liver' PROC. NATL. ACAD. SCI. USA vol. 83, no. 14, July 1986, pages 5311 - 5315, XP002945911
- KOMORI M. ET AL.: 'Molecular cloning and sequence analysis of cDNA containing the entire coding region for human fetal liver cytochrome P-450' J. BIOCHEM. vol. 105, no. 2, February 1989, TOKYO, pages 161 - 163, XP002945912
- PALMER C.N. ET AL.: 'Characterization of a cDNA encoding a human kidney, cytochrome P-450 4A fatty acid omega-hydroxylase and the cognate enzyme expressed in escherichia coli' BIOCHIM. BIOPHYS. ACTA vol. 1172, no. 1-2, 20 February 1993, pages 161 - 166, XP002945913
- NHAMBURO P.T. ET AL.: 'Identification of a new P450 expressed in human lung: complete cDNA sequence, cDNA-directed expression and chromosome mapping' BIOCHEMISTRY vol. 28, no. 20, 03 October 1989, pages 8060 - 8066, XP002945914
- KIKUTA Y. ET AL.: 'Cloning and expression of a novel form of leukotriene B4 omega-hydroxlase from human liver' FEBS LETT. vol. 348, no. 1, 04 July 1994, pages 70 - 74, XP002945915
- KIKUTA Y. ET AL.: 'A novel form of cytochrome P-450 family 4 in human polymorphonuclear leukocytes. cDNA cloning and expression of leukotriene B4 omega-hydroxylase' J. BIOL. CHEM. vol. 268, no. 13, 05 May 1993, pages 9376 - 9380, XP002945916
- MORNET E. ET AL.: 'Characterization of two genes encoding human steroid 11 beta-hydroxylase (P-450(II beta)' J. BIOL. CHEM. vol. 264, no. 35, 15 December 1989, pages 20961 - 20967, XP002945917
- CHUNG B.C. ET AL.: 'Cytochrome P450c17 (steroid 17 alpha-hydroxylase/17,20 lyase): cloning of human adrenal and testis cDNAs indicates the same gene is expressed in both tissues' PROC. NATL. ACAD. SCI. USA vol. 84, no. 2, January 1987, pages 407 - 411, XP002945918
- HARADA N. ET AL.: 'Cloning of a complete cDNA encoding human aromatase: immunochemical identification adn sequence analysis' BIOCHEM. BIOPHYS. RES. COMMUN. vol. 156, no. 2, 31 October 1988, pages 725 - 732, XP002945919
- CALI J.J. ET AL.: 'Characterization of human sterol 27-hydroxylase. A mitochondrial cytochrome P-450 that catalyzes multiple oxidation reactions in bile acid biosynthesis' J. BIOL. CHEM. vol. 266, no. 12, 25 April 1991, pages 7774 - 7778, XP002945920

### SEQUENCE LISTING

<110> Otsuka Pharmaceutical Factory Inc.
<120> A method for detecting human P450 molecular species and a prove and a kit therefor
<130> P01-38
<150> 2000-164214
<151> 2000-06-01.
<160> 105
<170> Patent In Ver. 2.0
<210> 1
   <211> 26
   <212> DNA
   <213> human P450 CYP1A1 gene
   <400> 1
   cgctatgacc acaaccacca agaact 26
<210> 2
   <211> 26
   <212> DNA
   <213> human P450 CYP1A2 gene
   <400> 2
   ctagagccag cggcaacctc atccca 26
<210> 3
   <211> 28
   <212> DNA
   <213> human P450 CYP1B1 gene
<400> 3
   cagctttgtg cctgtcacta ttcctcat 28
<210> 4
   <211> 24
   <212> DNA
   <213> human P450 CYP2A6/7 gene
   <400> 4
   tggccccgtg ttcaccattc actt 24
<210> 5
   <211> 30
   <212> DNA
   <213> human P450 CYP2A6 gene
   <400> 5
   tgcctgactg tgatggtctt gatgtctgtt 30
<210> 6
   <211> 25
   <212> DNA
   <213> human P450 CYP2A7 gene
   <400> 6
   aacctcttca ttgcaggcac cgaga 25
<210> 7
   <211> 28
   <212> DNA
   <213> human P450 CYP2B6 gene
   <400> 7
   tcatctgctc catcgtcttt ggaaaacg 28
<210> 8
   <211> 26
   <212> DNA
   <213> human P450 CYP2C8 gene
   <400> 8
   tttctccctc acaaccttgc ggaatt 26
<210> 9
   <211> 26
   <212> DNA
   <213> human P450 CYP2C9 gene
   <400> 9
   ttgtgcttgt cgtctctgtc ccagct 26
<210> 10
   <211> 25
   <212> DNA
   <213> human P450 CYP2C18 gene
   <400> 10
   aatgcatttg gtcgtgtgcc accct 25
<210> 11
   <211> 27
   <212> DNA
   <213> human P450 CYP2C19 gene
   <400> 11
   ttgtgcttgt tgtctctgtc ccagctc 27
<210> 12
   <211> 28
   <212> DNA
   <213> human P450 CYP2D6 gene
   <400> 12
   cagctggatg agctgctaac tgagcaca 28
<210> 13
   <211> 22
   <212> DNA
   <213> human P450 CYP2E1 gene
   <400> 13
   tccaacctgc cccatgaagc aa 22
<210> 14
   <211> 28
   <212> DNA
   <213> human P450 CYP2F1 gene
   <400> 14
   atgacaactt ccaaatcatg agcagccc 28
<210> 15
   <211> 24
   <212> DNA
   <213> human P450 CYP2J2 gene
   <400> 15
   aggcccaaca cctcactgaa gcaa 24
<210> 16
   <211> 30
   <212> DNA
   <213> human P450 CYP3A3/4 gene
   <400> 16
   tctggagctc gtggcccaat caattatctt 30
<210> 17
   <211> 29
   <212> DNA
   <213> human P450 CYP3A4 gene
   <400> 17
   aggagagaac actgctcgtg gtttcacag 29
<210> 18
   <211> 29
   <212> DNA
   <213> human P450 CYP3A5 gene
   <400> 18
   tttctttcga attctgggag tcaatcatc 29
<210> 19
   <211> 32
   <212> DNA
   <213> human P450 CYP3A7 gene
   <400> 19
   agtcttttga attctgagag tcaatcatca gc 32
<210> 20
   <211> 26
   <212> DNA
   <213> human P450 CYP4A11 gene
   <400> 20
   tgacctgaac aacctggttt tttccc 26
<210> 21
   <211> 28
   <212> DNA
   <213> human P450 CYP4B1 gene
   <400> 21
   ctgtaccctg agcaccagca tcgttgta 28
<210> 22
   <211> 24
   <212> DNA
   <213> human P450 CYP4F2 gene
   <400> 22
   agtcccggtc atctcccgcc atgt 24
<210> 23
   <211> 25
   <212> DNA
   <213> human P450 CYP4F3 gene
   <400> 23
   tgccgtctct cgctgctgca cccaa 25
<210> 24
   <211> 30
   <212> DNA
   <213> human P450 CYP4F8 gene
   <400> 24
   cgaaaacgcc cagaagaggt cacctatggc 30
<210> 25
   <211> 26
   <212> DNA
   <213> human P450 CYP11B1 gene
   <400> 25
   gcgcgtgttc ctctactctc tgggtc 26
<210> 26
   <211> 31
   <212> DNA
   <213> human P450 CYP11B2 gene
   <400> 26
   acaggttttc ctctactcgc tgggtcgcaa t 31
<210> 27
   <211> 26
   <212> DNA
   <213> human P450 CYP17 gene
   <400> 27
   tcagttcatg cctgagcgtt tettga 26
<210> 28
   <211> 29
   <212> DNA
   <213> human P450 CYP19 gene
   <400> 28
   ccttctttat gaaagctctg tcaggcccc 29
<210> 29
   <211> 28
   <212> DNA
   <213> human P450 CYP27 gene
   <400> 29
   tgcgcttctt ctttcagctg ttcgttca 28
<210> 30
   <211> 28
   <212> DNA
   <213> human P450 CYP1B1 gene
   <400> 30
   agcagctcaa ccgcaacttc agcaactt 28
<210> 31
   <211> 28
   <212> DNA
   <213> human P450 CYP2B6 gene
   <400> 31
   tgagcactgc tctccatgac ccacacta 28
<210> 32
   <211> 32
   <212> DNA
   <213> human P450 CYP2C8 gene
   <400> 32
   ttggcactgt agctgatcta tttgttgctg ga 32
<210> 33
   <211> 28
   <212> DNA
   <213> human P450 CYP2C9 gene
   <400> 33
   aaaacactgc agttgacttg tttggagc 28
<210> 34
   <211> 32
   <212> DNA
   <213> human P450 CYP2C19 gene
   <400> 34
   taatcactgc agctgactta cttggagctg gg 32
<210> 35
   <211> 26
   <212> DNA
   <213> human P450 CYP4C11 gene
   <400> 35
   acattcccaa gtgcctgtcc tcattg 26
<210> 36
   <211> 22
   <212> DNA
   <213> human P450 CYP1A1 gene
   <400> 36
   gtcatctgtg ccatttgctt tg 22
<210> 37
   <211> 22
   <212> DNA
   <213> human P450 CYP1A1 gene
   <400> 37
   caaccacctc cccgaaatta tt 22
<210> 38
   <211> 21
   <212> DNA
   <213> human P450 CYP1A2 gene
   <400> 38
   tgttcaagca cagcaagaag g 21
<210> 39
   <211> 21
   <212> DNA
   <213> human P450 CYP1A2 gene
   <400> 39
   tgctccaaag acgtcattga c 21
<210> 40
   <211> 20
   <212> DNA
   <213> human P450 CYP1B1 gene
   <400> 40
   ttatgaagcc atgcgcttct 20
<210> 41
   <211> 22
   <212> DNA
   <213> human P450 CYP1B1 gene
   <400> 41
   agacagaggt gttggcagtg gt 22
<210> 42
   <211> 21
   <212> DNA
   <213> human P450 CYP2A6/7 gene
   <400> 42
   tcatgaagat cagtgagcgc t 21
<210> 43
   <211> 19
   <212> DNA
   <213> human P450 CYP2A6/7 gene
   <400> 43
   tcatgtccac acagcacca 19
<210> 44
   <211> 19
   <212> DNA
   <213> human P450 CYP2A6 gene
   <400> 44
   ttttggtggc cttgctggt 19
<210> 45
   <211> 26
   <212> DNA
   <213> human P450 CYP2A6 gene
   <400> 45
   ggagttgtac atctgctctg tgttca 26
<210> 46
   <211> 24
   <212> DNA
   <213> human P450 CYP2A7 gene
   <400> 46
   cttgaagaac ctgatgatga gcac 24
<210> 47
   <211> 23
   <212> DNA
   <213> human P450 CYP2A7 gene
   <400> 47
   ctctgtcaat ctcctcatgg acc 23
<210> 48
   <211> 19
   <212> DNA
   <213> human P450 CYP2B6 gene
   <400> 48
   ccattccatt accgccaac 19
<210> 49
   <211> 24
   <212> DNA
   <213> human P450 CYP2B6 gene
   <400> 49
   aggaactctt gatcttggta gtgg 24
<210> 50
   <211> 21
   <212> DNA
   <213> human P450 CYP2C8 gene
   <400> 50
   aagagatgga aggagatccg g 21
<210> 51
   <211> 19
   <212> DNA
   <213> human P450 CYP2C8 gene
   <400> 51
   tcaatgctcc tcttcccca 19
<210> 52
   <211> 24
   <212> DNA
   <213> human P450 CYP2C9 gene
   <400> 52
   ctcctatcat tgattacttc ccgg 24
<210> 53
   <211> 23
   <212> DNA
   <213> human P450 CYP2C9 gene
   <400> 53
   ggagaaggag agcatatctc agg 23
<210> 54
   <211> 21
   <212> DNA
   <213> human P450 CYP2C18 gene
   <400> 54
   aggatattga catcaccccc a 21
<210> 55
   <211> 23
   <212> DNA
   <213> human P450 CYP2C18 gene
   <400> 55
   tcagacagga atgaagcaga get 23
<210> 56
   <211> 23
   <212> DNA
   <213> human P450 CYP2C19 gene
   <400> 56
   cttggtaatc actgcagctg act 23
<210> 57
   <211> 23
   <212> DNA
   <213> human P450 CYP2C19 gene
   <400> 57
   tcagcaggag aaggagagca tat 23
<210> 58
   <211> 21
   <212> DNA
   <213> human P450 CYP2D6 gene
   <400> 58
   cctacgcttc caaaaggctt t 21
<210> 59
   <211> 22
   <212> DNA
   <213> human P450 CYP2D6 gene
   <400> 59
   agagaacagg tcagccacca ct 22
<210> 60
   <211> 19
   <212> DNA
   <213> human P450 CYP2E1 gene
   <400> 60
   ttcagcggtt catcaccct 19
<210> 61
   <211> 24
   <212> DNA
   <213> human P450 CYP2E1 gene
   <400> 61
   gaggtatcct ctgaaaatgg tgtc 24
<210> 62
   <211> 22
   <212> DNA
   <213> human P450 CYP2F1 gene
   <400> 62
   gctcaccatt atccgcctta tc 22
<210> 63
   <211> 22
   <212> DNA
   <213> human P450 CYP2F1 gene
   <400> 63
   gaggtctctc aggcacttga ag 22
<210> 64
   <211> 23
   <212> DNA
   <213> human P450 CYP2J2 gene
   <400> 64
   agcttagagg aacgcattca gga 23
<210> 65
   <211> 22
   <212> DNA
   <213> human P450 CYP2J2 gene
   <400> 65
   cgaaggtgat ggagcaaatg at 22
<210> 66
   <211> 23
   <212> DNA
   <213> human P450 CYP3A3/4 gene
   <400> 66
   actgagtccc acaaagctct gtc 23
<210> 67
   <211> 22
   <212> DNA
   <213> human P450 CYP3A3/4 gene
   <400> 67
   aactgcatca atttcctcct gc 22
<210> 68
   <211> 30
   <212> DNA
   <213> human P450 CYP3A4 gene
   <400> 68
   gattgactct cagaattcaa aagaaactga 30
<210> 69
   <211> 26
   <212> DNA
   <213> human P450 CYP3A4 gene
   <400> 69
   ggtgagtggc cagttcatac ataatg 26
<210> 70
   <211> 22
   <212> DNA
   <213> human P450 CYP3A5 gene
   <400> 70
   ccttacccca gtttttgaag ca 22
<210> 71
   <211> 23
   <212> DNA
   <213> human P450 CYP3A5 gene
   <400> 71
   tccagatcag acagagcttt gtg 23
<210> 72
   <211> 22
   <212> DNA
   <213> human P450 CYP3A7 gene
   <400> 72
   ccttacccca attcttgaag ca 22
<210> 73
   <211> 23
   <212> DNA
   <213> human P450 CYP3A7 gene
   <400> 73
   tccagatcag acagagcttt gtg 23
<210> 74
   <211> 24
   <212> DNA
   <213> human P450 CYP4A11 gene
   <400> 74
   tctcagtcct acatacaggc catt 24
<210> 75
   <211> 22
   <212> DNA
   <213> human P450 CYP4A11 gene
   <400> 75
   gttggatcac ttggtctgtg tg 22
<210> 76
   <211> 22
   <212> DNA
   <213> human P450 CYP4B1 gene
   <400> 76
   cctggtttct ctactgcatg gc 22
<210> 77
   <211> 21
   <212> DNA
   <213> human P450 CYP4B1 gene
   <400> 77
   ccagatcatc ccactggaag a 21
<210> 78
   <211> 22
   <212> DNA
   <213> human P450 CYP4F2 gene
   <400> 78
   ccgtgagcct aaagagattg aa 22
<210> 79
   <211> 21
   <212> DNA
   <213> human P450 CYP4F2 gene
   <400> 79
   cgaaaacact gatgaggcag a 21
<210> 80
   <211> 22
   <212> DNA
   <213> human P450 CYP4F3 gene
   <400> 80
   tacaagagct tctgaaggac cg 22
<210> 81
   <211> 22
   <212> DNA
   <213> human P450 CYP4F3 gene
   <400> 81
   tgatgaggca gataatgcct tt 22
<210> 82
   <211> 22
   <212> DNA
   <213> human P450 CYP4F8 gene
   <400> 82
   aatccatcac aacccctcag tc 22
<210> 83
   <211> 20
   <212> DNA
   <213> human P450 CYP4F8 gene
   <400> 83
   ccgccgagaa aggaataaaa 20
<210> 84
   <211> 22
   <212> DNA
   <213> human P450 CYP11B1 gene
   <400> 84
   gagctcagac ttggtgcttc ag 22
<210> 85
   <211> 20
   <212> DNA
   <213> human P450 CYP11B1 gene
   <400> 85
   tggaggtgtt tcagcacatg 20
<210> 86
   <211> 23
   <212> DNA
   <213> human P450 CYP11B2 gene
   <400> 86
   cttggtgctt cagaactacc aca 23
<210> 87
   <211> 23
   <212> DNA
   <213> human P450 CYP11B2 gene
   <400> 87
   ttagtgtctc caccaggaag tgc 23
<210> 88
   <211> 22
   <212> DNA
   <213> human P450 CYP17 gene
   <400> 88
   tcacaatgag aaggagtggc ac 22
<210> 89
   <211> 23
   <212> DNA
   <213> human P450 CYP17 gene
   <400> 89
   agcttactga cggtgagatg agc 23
<210> 90
   <211> 22
   <212> DNA
   <213> human P450 CYP19 gene
   <400> 90
   agagctctgg aaaacaactc ga 22
<210> 91
   <211> 21
   <212> DNA
   <213> human P450 CYP19 gene
<400> 91
   ctgtgaccat acgaacaagg c 21
<210> 92
   <211> 23
   <212> DNA
   <213> human P450 CYP27 gene
   <400> 92
   agaggagatt ccacgtctag gac 23
<210> 93
   <211> 22
   <212> DNA
   <213> human P450 CYP27 gene
   <400> 93
   acatccacat tggaccgtac tt 22
<210> 94
   <211> 18
   <212> DNA
   <213> human P450 CYP1B1 gene
   <400> 94
   accgttttcc gcgaattc 18
<210> 95
   <211> 21
   <212> DNA
   <213> human P450 CYP1B1 gene
   <400> 95
   gtacgttctc caaatccagc c 21
<210> 96
   <211> 23
   <212> DNA
   <213> human P450 CYP2B6 gene
   <400> 96
   ccccaaggac acagaagtat ttc 23
<210> 97
   <211> 22
   <212> DNA
   <213> human P450 CYP2B6 gene
   <400> 97
   gattgaaggc gtctggtttt tc 22
<210> 98
   <211> 22
   <212> DNA
   <213> human P450 CYP2C8 gene
   <400> 98
   ggactttatc gattgcttcc tg 22
<210> 99
   <211> 22
   <212> DNA
   <213> human P450 CYP2C8 gene
   <400> 99
   ccatatctca gagtggtgct tg 22
<210> 100
   <211> 24
   <212> DNA
   <213> human P450 CYP2C9 gene
   <400> 100
   gacatgaaca accctcagga cttt 24
<210> 101
   <211> 20
   <212> DNA
   <213> human P450 CYP2C9 gene
   <400> 101
   tgcttgtcgt ctctgtccca 20
<210> 102
   <211> 22
   <212> DNA
   <213> human P450 CYP2C19 gene
   <400> 102
   gaacaccaag aatcgatgga ca 22
<210> 103
   <211> 22
   <212> DNA
   <213> human P450 CYP2C19 gene
   <400> 103
   tcagcaggag aaggagagca ta 22
<210> 104
   <211> 22
   <212> DNA
   <213> human P450 CYP4A11 gene
   <400> 104
   aggagctaca acggattcag aa 22
<210> 105
   <211> 21
   <212> DNA
   <213> human P450 CYP4A11 gene
   <400> 105
   acgaactttg cctccccata g 21

### SEQUENCE LISTING

<110> Otsuka Pharmaceutical Factory Inc.
   <120> A method for detecting human P450 molecular species and a prove and a kit therefor
   <130> P01-38
   <150> 2000-164214
   <151> 2000-06-01
   <160> 105
   <170> PatentIn Ver. 2.0
<210> 1
   <211> 26
   <212> DNA
   <213> human P450 CYP1A1 gene
   <400> 1
   cgctatgacc acaaccacca agaact 26
<210> 2
   <211> 26
   <212> DNA
   <213> human P450 CYP1A2 gene
   <400> 2
   ctagagccag cggcaacctc atccca 26
<210> 3
   <211> 28
   <212> DNA
   <213> human P450 CYP1B1 gene
<400> 3
   cagctttgtg cctgtcacta ttcctcat 28
<210> 4
   <211> 24
   <212> DNA
   <213> human P450 CYP2A6/7 gene
   <400> 4
   tggccccgtg ttcaccattc actt 24
<210> 5
   <211> 30
   <212> DNA
   <213> human P450 CYP2A6 gene
   <400> 5
   tgcctgactg tgatggtctt gatgtctgtt 30
<210> 6
   <211> 25
   <212> DNA
   <213> human P450 CYP2A7 gene
   <400> 6
   aacctcttca ttgcaggcac cgaga 25
<210> 7
   <211> 28.
   <212> DNA
   <213> human P450 CYP2B6 gene
   <400> 7
   tcatctgctc catcgtcttt ggaaaacg 28
<210> 8
   <211> 26
   <212> DNA
   <213> human P450 CYP2C8 gene
   <400> 8
   tttctccctc acaaccttgc ggaatt 26
<210> 9
   <211> 26
   <212> DNA
   <213> human P450 CYP2C9 gene
   <400> 9
   ttgtgcttgt cgtctctgtc ccagct 26
<210> 10
   <211> 25
   <212> DNA
   <213> human P450 CYP2C18 gene
   <400> 10
   aatgcatttg gtcgtgtgcc accct 25
<210> 11
   <211> 27
   <212> DNA
   <213> human P450 CYP2C19 gene
   <400> 11
   ttgtgcttgt tgtctctgtc ccagctc 27
<210> 12
   <211> 28
   <212> DNA
   <213> human P450 CYP2D6 gene
   <400> 12
   cagctggatg agctgctaac tgagcaca 28
<210> 13
   <211> 22
   <212> DNA
   <213> human P450 CYP2E1 gene
   <400> 13
   tccaacctgc cccatgaagc aa 22
<210> 14
   <211> 28
   <212> DNA
   <213> human P450 CYP2F1 gene
   <400> 14
   atgacaactt ccaaatcatg agcagccc 28
<210> 15
   <211> 24
   <212> DNA
   <213> human P450 CYP2J2 gene
   <400> 15
   aggcccaaca cctcactgaa gcaa 24
<210> 16
   <211> 30
   <212> DNA
   <213> human P450 CYP3A3/4 gene
   <400> 16
   tctggagctc gtggcccaat caattatctt 30
<210> 17
   <211> 29
   <212> DNA
   <213> human P450 CYP3A4 gene
   <400> 17
   aggagagaac actgctcgtg gtttcacag 29
<210> 18
   <211> 29
   <212> DNA
   <213> human P450 CYP3A5 gene
   <400> 18
   tttctttcga attctgggag tcaatcatc 29
<210> 19
   <211> 32
   <212> DNA
   <213> human P450 CYP3A7 gene
   <400> 19
   agtcttttga attctgagag tcaatcatca gc 32
<210> 20
   <211> 26
   <212> DNA
   <213> human P450 CYP4A11 gene
   <400> 20
   tgacctgaac aacctggttt tttccc 26
<210> 21
   <211> 28
   <212> DNA
   <213> human P450 CYP4B1 gene
   <400> 21
   ctgtaccctg agcaccagca tcgttgta 28
<210> 22
   <211> 24
   <212> DNA
   <213> human P450 CYP4F2 gene
   <400> 22
   agtcccggtc atctcccgcc atgt 24
<210> 23
   <211> 25
   <212> DNA
   <213> human P450 CYP4F3 gene
   <400> 23
   tgccgtctct cgctgctgca cccaa 25
<210> 24
   <211> 30
   <212> DNA
   <213> human P450 CYP4F8 gene
   <400> 24
   cgaaaacgcc cagaagaggt cacctatggc 30
<210> 25
   <211> 26
   <212> DNA
   <213> human P450 CYP11B1 gene
   <400> 25
   gcgcgtgttc ctctactctc tgggtc 26
<210> 26
   <211> 31
   <212> DNA
   <213> human P450 CYP11B2 gene
   <400> 26
   acaggttttc ctctactcgc tgggtcgcaa t 31
<210> 27
   <211> 26
   <212> DNA
   <213> human P450 CYP17 gene
   <400> 27
   tcagttcatg cctgagcgtt tcttga 26
<210> 28
   <211> 29
   <212> DNA
   <213> human P450 CYP19 gene
   <400> 28
   ccttctttat gaaagctctg tcaggcccc 29
<210> 29
   <211> 28
   <212> DNA
   <213> human P450 CYP27 gene
   <400> 29
   tgcgcttctt ctttcagctg ttcgttca 28
<210> 30
   <211> 28
   <212> DNA
   <213> human P450 CYP1B1 gene
   <400> 30
   agcagctcaa ccgcaacttc agcaactt 28
<210> 31
   <211> 28
   <212> DNA
   <213> human P450 CYP2B6 gene
   <400> 31
   tgagcactgc tctccatgac ccacacta 28
<210> 32
   <211> 32
   <212> DNA
   <213> human P450 CYP2C8 gene
   <400> 32
   ttggcactgt agctgatcta tttgttgctg ga 32
<210> 33
   <211> 28
   <212> DNA
   <213> human P450 CYP2C9 gene
   <400> 33
   aaaacactgc agttgacttg tttggagc 28
<210> 34
   <211> 32
   <212> DNA
   <213> human P450 CYP2C19 gene
   <400> 34
   taatcactgc agctgactta cttggagctg gg 32
<210> 35
   <211> 26
   <212> DNA
   <213> human P450 CYP4C11 gene
   <400> 35
   acattcccaa gtgcctgtcc tcattg 26
<210> 36
   <211> 22
   <212> DNA
   <213> human P450 CYP1A1 gene
   <400> 36
   gtcatctgtg ccatttgctt tg 22
<210> 37
   <211> 22
   <212> DNA
   <213> human P450 CYP1A1 gene
   <400> 37
   caaccacctc cccgaaatta tt 22
<210> 38
   <211> 21
   <212> DNA
   <213> human P450 CYP1A2 gene
   <400> 38
   tgttcaagca cagcaagaag g 21
<210> 39
   <211> 21
   <212> DNA
   <213> human P450 CYP1A2 gene
   <400> 39
   tgctccaaag acgtcattga c 21
<210> 40
   <211> 20
   <212> DNA
   <213> human P450 CYP1B1 gene
   <400> 40
   ttatgaagcc atgcgcttct 20
<210> 41
   <211> 22
   <212> DNA
   <213> human P450 CYP1B1 gene
   <400> 41
   agacagaggt gttggcagtg gt 22
<210> 42
   <211> 21
   <212> DNA
   <213> human P450 CYP2A6/7 gene
   <400> 42
   tcatgaagat cagtgagcgc t 21
<210> 43
   <211> 19
   <212> DNA
   <213> human P450 CYP2A6/7 gene
   <400> 43
   tcatgtccac acagcacca 19
<210> 44
   <211> 19
   <212> DNA
   <213> human P450 CYP2A6 gene
   <400> 44
   ttttggtggc cttgctggt 19
<210> 45
   <211> 26
   <212> DNA
   <213> human P450 CYP2A6 gene
   <400> 45
   ggagttgtac atctgctctg tgttca **2**6
<210> 46
   <211> 24
   <212> DNA
   <213> human P450 CYP2A7 gene
   <400> 46
   cttgaagaac ctgatgatga gcac 24
<210> 47
   <211> 23
   <212> DNA
   <213> human P450 CYP2A7 gene
   <400> 47
   ctctgtcaat ctcctcatgg acc 23
<210> 48
   <211> 19
   <212> DNA
   <213> human P450 CYP2B6 gene
   <400> 48
   ccattccatt accgccaac 19
<210> 49
   <211> 24
   <212> DNA
   <213> human P450 CYP2B6 gene
   <400> 49
   aggaactctt gatcttggta gtgg 24
<210> 50
   <211> 21
   <212> DNA
   <213> human P450 CYP2C8 gene
   <400> 50
   aagagatgga aggagatccg g 21
<210> 51
   <211> 19
   <212> DNA
   <213> human P450 CYP2C8 gene
   <400> 51
   tcaatgctcc tcttcccca 19
<210> 52
   <211> 24
   <212> DNA
   <213> human P450 CYP2C9 gene
   <400> 52
   ctcctatcat tgattacttc ccgg 24
<210> 53
   <211> 23
   <212> DNA
   <213> human P450 CYP2C9 gene
   <400> 53
   ggagaaggag agcatatctc agg 23
<210> 54
   <211> 21
   <212> DNA
   <213> human P450 CYP2C18 gene
   <400> 54
   aggatattga catcaccccc a 21
<210> 55
   <211> 23
   <212> DNA
   <213> human P450 CYP2C18 gene
   <400> 55
   tcagacagga atgaagcaga gct 23
<210> 56
   <211> 23
   <212> DNA
   <213> human P450 CYP2C19 gene
   <400> 56
   cttggtaatc actgcagctg act 23
<210> 57
   <211> 23
   <212> DNA
   <213> human P450 CYP2C19 gene
   <400> 57
   tcagcaggag aaggagagca tat 23
<210> 58
   <211> 21
   <212> DNA
   <213> human P450 CYP2D6 gene
   <400> 58
   cctacgcttc caaaaggctt t 21
<210> 59
   <211> 22
   <212> DNA
   <213> human P450 CYP2D6 gene
   <400> 59
   agagaacagg tcagccacca ct 22
<210> 60
   <211> 19
   <212> DNA
   <213> human P450 CYP2E1 gene
   <400> 60
   ttcagcggtt catcaccct 19
<210> 61
   <211> 24
   <212> DNA
   <213> human P450 CYP2E1 gene
   <400> 61
   gaggtatcct ctgaaaatgg tgtc 24
<210> 62
   <211> 22
   <212> DNA
   <213> human P450 CYP2F1 gene
   <400> 62
   gctcaccatt atccgcctta tc 22
<210> 63
   <211> 22
   <212> DNA
   <213> human P450 CYP2F1 gene
   <400> 63
   gaggtctctc aggcacttga ag 22
<210> 64
   <211> 23
   <212> DNA
   <213> human P450 CYP2J2 gene
   <400> 64
   agcttagagg aacgcattca gga 23
<210> 65
   <211> 22
   <212> DNA
   <213> human P450 CYP2J2 gene
   <400> 65
   cgaaggtgat ggagcaaatg at 22
<210> 66
   <211> 23
   <212> DNA
   <213> human P450 CYP3A3/4 gene
   <400> 66
   actgagtccc acaaagctct gtc 23
<210> 67
   <211> 22
   <212> DNA
   <213> human P450 CYP3A3/4 gene
   <400> 67
   aactgcatca atttcctcct gc 22
<210> 68
   <211> 30
   <212> DNA
   <213> human P450 CYP3A4 gene
   <400> 68
   gattgactct cagaattcaa aagaaactga 30
<210> 69
   <211> 26
   <212> DNA
   <213> human P450 CYP3A4 gene
   <400> 69
   ggtgagtggc cagttcatac ataatg 26
<210> 70
   <211> 22
   <212> DNA
   <213> human P450 CYP3A5 gene
   <400> 70
   ccttacccca gtttttgaag ca 22
<210> 71
   <211> 23
   <212> DNA
   <213> human P450 CYP3A5 gene
   <400> 71
   tccagatcag acagagcttt gtg 23
<210> 72
   <211> 22
   <212> DNA
   <213> human P450 CYP3A7 gene
   <400> 72
   ccttacccca attcttgaag ca 22
<210> 73
   <211> 23
   <212> DNA
   <213> human P450 CYP3A7 gene
   <400> 73
   tccagatcag acagagcttt gtg 23
<210> 74
   <211> 24
   <212> DNA
   <213> human P450 CYP4A11 gene
   <400> 74
   tctcagtcct acatacaggc catt 24
<210> 75
   <211> 22
   <212> DNA
   <213> human P450 CYP4A11 gene
   <400> 75
   gttggatcac ttggtctgtg tg 22
<210> 76
   <211> 22
   <212> DNA
   <213> human P450 CYP4B1 gene
   <400> 76
   cctggtttct ctactgcatg gc 22
<210> 77
   <211> 21
   <212> DNA
   <213> human P450 CYP4B1 gene
   <400> 77
   ccagatcatc ccactggaag a 21
<210> 78
   <211> 22
   <212> DNA
   <213> human P450 CYP4F2 gene
   <400> 78
   ccgtgagcct aaagagattg aa 22
<210> 79
   <211> 21
   <212> DNA
   <213> human P450 CYP4F2 gene
   <400> 79
   cgaaaacact gatgaggcag a 21
<210> 80
   <211> 22
   <212> DNA
   <213> human P450 CYP4F3 gene
   <400> 80
   tacaagagct tctgaaggac cg 22
<210> 81
   <211> 22
   <212> DNA
   <213> human P450 CYP4F3 gene
   <400> 81
   tgatgaggca gataatgcct tt 22
<210> 82
   <211> 22
   <212> DNA
   <213> human P450 CYP4F8 gene
   <400> 82
   aatccatcac aacccctcag tc 22
<210> 83
   <211> 20
   <212> DNA
   <213> human P450 CYP4F8 gene
   <400> 83
   ccgccgagaa aggaataaaa 20
<210> 84
   <211> 22
   <212> DNA
   <213> human P450 CYP11B1 gene
   <400> 84
   gagctcagac ttggtgcttc ag 22
<210> 85
   <211> 20
   <212> DNA
   <213> human P450 CYP11B1 gene
   <400> 85
   tggaggtgtt tcagcacatg 20
<210> 86
   <211> 23
   <212> DNA
   <213> human P450 CYP11B2 gene
   <400> 86
   cttggtgctt cagaactacc aca 23
<210> 87
   <211> 23
   <212> DNA
   <213> human P450 CYP11B2 gene
   <400> 87
   ttagtgtctc caccaggaag tgc 23
<210> 88
   <211> 22
   <212> DNA
   <213> human P450 CYP17 gene
   <400> 88
   tcacaatgag aaggagtggc ac 22
<210> 89
   <211> 23
   <212> DNA
   <213> human P450 CYP17 gene
   <400> 89
   agcttactga cggtgagatg agc 23
<210> 90
   <211> 22
   <212> DNA
   <213> human P450 CYP19 gene
<400> 90
   agagctctgg aaaacaactc ga 22
<210> 91
   <211> 21
   <212> DNA
   <213> human P450 CYP19 gene
   <400> 91
   ctgtgaccat acgaacaagg c 21
<210> 92
   <211> 23
   <212> DNA
   <213> human P450 CYP27 gene
   <400> 92
   agaggagatt ccacgtctag gac 23
<210> 93
   <211> 22
   <212> DNA
   <213> human P450 CYP27 gene
   <400> 93
   acatccacat tggaccgtac tt 22
<210> 94
   <211> 18
   <212> DNA
   <213> human P450 CYP1B1 gene
   <400> 94
   accgttttcc gcgaattc 18
<210> 95
   <211> 21
   <212> DNA
   <213> human P450 CYP1B1 gene
   <400> 95
   gtacgttctc caaatccagc c 21
<210> 96
   <211> 23
   <212> DNA
   <213> human P450 CYP2B6 gene
   <400> 96
   ccccaaggac acagaagtat ttc 23
<210> 97
   <211> 22
   <212> DNA
   <213> human P450 CYP2B6 gene
   <400> 97
   gattgaaggc gtctggtttt tc 22
<210> 98
   <211> 22
   <212> DNA
   <213> human P450 CYP2C8 gene
   <400> 98
   ggactttatc gattgcttcc tg 22
<210> 99
   <211> 22
   <212> DNA
   <213> human P450 CYP2C8 gene
   <400> 99
   ccatatctca gagtggtgct tg 22
<210> 100
   <211> 24
   <212> DNA
   <213> human P450 CYP2C9 gene
   <400> 100
   gacatgaaca accctcagga cttt 24
<210> 101
   <211> 20
   <212> DNA
   <213> human P450 CYP2C9 gene
   <400> 101
   tgcttgtcgt ctctgtccca 20
<210> 102
   <211> 22
   <212> DNA
   <213> human P450 CYP2C19 gene
   <400> 102
   gaacaccaag aatcgatgga ca 22
<210> 103
   <211> 22
   <212> DNA
   <213> human P450 CYP2C19 gene
   <400> 103
   tcagcaggag aaggagagca ta 22
<210> 104
   <211> 22
   <212> DNA
   <213> human P450 CYP4A11 gene
   <400> 104
   aggagctaca acggattcag aa 22
<210> 105
   <211> 21
   <212> DNA
   <213> human P450 CYP4A11 gene
   <400> 105
   acgaactttg cctccccata g 21

## Description

### TECHNICAL FIELD

The present invention relates to a method of detecting and quantifying the CYP1A1 gene of the human P450 molecular species, and a kit for use in the method.

### BACKGROUND OF THE INVENTION

Cytochrome P450 is one of the enzymes that participate in the metabolism of chemical substances, such as active ingredient compounds in medicines. A number of molecular species of cytochrome P450 are known to exist, and about 30 molecular species have been hitherto confirmed in humans. Although these molecular species are different in enzyme activity from one another, they are each reported to participate in the metabolism of active ingredient compounds in medicines (e.g., active ingredient compounds in tricyclic antidepressants, antiepileptics, benzodiazepine preparations, β-blockers, barbital sleep-inducing hypnotics, and other medicines, dimethylnitrosamine, etc.), benzene and other organic solvents, low-molecular-weight carcinogens in the environment, or the like (for example, Yoo, J.S.H., Chung, R., C., Wade, D., & Yang, C.S.(1987) Cancer Res., 47, 3378-3383).

In the development of a new medicine, it is important to understand the actions (changes in expression levels) of molecular species of human cytochrome P450 (hereinafter referred to simply as "P450") upon administration of the new medicine. This is because, if the changes in the expression levels of P450 molecular species are unknown, it is impossible to predict an increase or decrease in efficacy and side effects of the new medicine caused by a medicine or another substance concurrently administered with the new medicine, or an increase or decrease in efficacy and side effects of the medicine concurrently administered. Thus, the safety of the new medicine cannot be confirmed.

Therefore, in pharmaceutical fields, such as new medicine development, information concerning P450 molecular species, and in particular, a technique for measuring the levels of P450 molecular species individually to obtain information concerning each P450 molecular species, have been desired. The development of a technique for measuring the molecular species individually would make it possible to, for example, easily understand the interaction of a new medicine with another medicine and the influence of the new medicine on organisms in a special morbid condition, and to acquire information about the side effects of the new medicine, thereby ensuring its safety.

The polymerase chain reaction (PCR) is a widely known method for amplifying nucleic acids. Of the PCR techniques, RT-PCR (Reverse Transcription-PCR), competitive RT-PCR and the like are used for detecting and quantifying a trace amount of mRNA, and show their effectiveness.

In recent years, a real-time quantitative detection technique using PCR has been established (TaqMan PCR, Genome Res., 6 (10), 986 (1996), ABI PRISM^{™} Sequence Detection System, Applied Biosystems). This technique measures the amount of nucleic acids using a particular fluorescent-labeled probe (TaqMan probe). More specifically, this technique utilizes the following principles: For example, a fluorescent-labeled probe having a reporter dye at the 5' end and a quencher dye at the 3' end is annealed to the target DNA, and the DNA is subjected to normal PCR. As the extension reaction proceeds, the probe is hydrolyzed from the 5' end by the 5'-3' exonuclease activity possessed by DNA polymerase. As a result, the reporter dye at the 5' end is separated from the quencher dye at the 3' end, thereby eliminating the FRET (Fluorescence Resonance Energy Transfer, the reduction in fluorescence intensity owing to the decrease in the energy level of the reporter dye caused by the resonance of the two fluorescent dyes) effect produced by the spatial proximity between the two dyes, and increasing the fluorescence intensity of the reporter dye that has been controlled by the quencher dye. The target nucleic acid can be selectively quantified and detected in real-time by measuring the increase of the fluorescence intensity.

This technique is advantageous in that it can test various samples simultaneously in a short time, since, unlike the detection and quantification technique using conventional PCR it does not involve complicated steps, such as agarose gel electrophoresis of the amplified product after PCR and analysis of the electrophoresis pattern.

Generally, when conducting clinical tests in a clinical test center or the like, it is necessary to inspect an extremely large number of samples within a limited time. Therefore, there is considerable demand for the development of efficient test techniques. The real-time quantitative detection technique is a promising candidate to meet this demand.

The present inventors turned their attention to the real-time quantitative detection technique using PCR, and conceived that, if the detection technique can be utilized for detecting human P450 molecular species, the molecular species can be individually detected and quantified using the same apparatus under the same PCR conditions.

However, as described above, there are about 30 presently known P450 molecular species participating in the metabolism of chemical substances. Although the mRNA sequence of each species is known, it was deemed to be difficult to find oligonucleotides (for use as primer pairs) that do not overlap with one another and are capable of sufficiently amplifying these molecular species as target genes and hybridizing with particular portions of the target genes. It has been also assumed difficult to constitute, in a specific region between the primer pair, a probe that is capable of hybridizing with the target gene faster than the primers under the same PCR conditions, and is specific to only one molecular species. In particular, it is known that, although the ease of hybridization between two nucleic acids of known nucleotide sequences can be estimated to a certain extent by calculating the melting point (Tm), the combination of primers and a probe selected based on the estimation does not necessarily bring good results in DNA measurement. Therefore, many trial-and-error experiments by experts are expected to be required to select, for each of all the presently known P450 molecular species, a combination of a primer pair and probe for real-time PCR detection which is capable of measuring its gene individually.

### DISCLOSURE OF THE INVENTION

An object of the present invention is to provide a method of detecting and quantifying the CYP1A1 gene encoding a P450 molecular species, and especially a method of detecting and quantifying the gene by the real-time quantitative detection technique.

Another object of the present invention is to provide probes and primer pairs for use in the above method.

In order to achieve the above objects, the present inventors repeatedly conducted a great number of experiments and research on P450 molecular species. As a result, the present inventors succeeded in finding sets consisting of a primer pair and a probe that are specific to 29 particular genes of P450 molecular species, i.e., sets of a primer pair and a probe that are capable of individually detecting and quantifying the genes encoding the molecular species. The present invention has been accomplished based on this finding.

The present invention provides a kit for detecting and quantifying the CYP1A1 gene of the human cytochrom P450 molecular species, comprising a set of a primer pair of a forward primer comprising an oligonucleotide which hybridizes with the 589-610 region of the CYP1A1 gene and a reverse primer comprising an oligonucleotide which hybridizes with the 685-664 region of the CYP1A1 gene, and a probe comprising an oligonucleotide which hybridizes with the 616-641 region of the CYP1A1 gene.

More specifically, the present invention provides the above kit in which the probe further comprises a reporter dye and a quencher dye both attached to the oligonucleotide.

Preferably, the set of a primer pair and a probe in the above kit is a set of a primer pair comprising the sequences shown in SEQ ID NOS: 36 and 37 and a probe comprising the sequence shown in SEQ ID NO: 1

The above set is used for detecting and quantifying the following P450 molecular species.

The present invention further provides a method of detecting and quantifying the CYP1A1 gene of the P450 molecular species, the method comprising the steps (a) to (c) described below, in particular, the method wherein, in step (c), the hydrolyzed probe or probes are detected and quantified by detecting the fluorescence produced by irradiation with excitation light, and measuring the degree of fluorescence:
(a) preparing a sample containing a P450 gene or genes;
(b) subjecting the sample to polymerase chain reaction (PCR, which may be RT-PCR) using a kit of the present invention comprising the above set of a primer pair and a probe to amplify the CYP1A1 gene encoding the P450 molecular species in the sample; and
(c) detecting and measuring the probe or probes hydrolyzed during the polymerase chain reaction.

In this specification, the abbreviations for amino acids, peptides, nucleotide sequences, nucleic acids, etc., are those provided in the IUPAC-IUB Communication on Biological Nomenclature, Eur. J. Biochem., 138: 9 (1984) or "Guideline for preparation of a specification containing nucleotide sequences or amino acid sequences" (JPO), or those conventionally used in this field.

The term "gene" in this specification includes double-stranded DNA and single-stranded sense or antisense DNA constituting double-stranded DNA. The term further includes mRNA involved in the expression of these genes, and cDNA complementary to the mRNA. The term "nucleotide" ("oligonucleotide") includes RNA and DNA.

It is more preferable to perform the method of the present invention according to the real-time detection technique using a probe labeled with a reporter dye and a quencher dye.

The real-time detection technique is capable of easily and rapidly quantifying the genes of molecular species individually in real-time, under the same PCR or RT-PCR conditions, and the establishment of this technique is very beneficial to the clinical and pharmaceutical fields. For example, the technique can easily quantify the P450 mRNA expression in a human sample, which is important in kinetics tests for the development of medicines, thereby making it possible to know the activity of a P450 molecular species when exposed to a chemical substance, such as a medicine. In particular, in the development of new medicines, researchers are required to investigate the activity of the metabolism by P450. Conventionally, the activity of the metabolism by P450 has been investigated by measuring the expression level of each P450 molecular species separately, by a complicated, labor- and time-consuming process. In contrast, the present invention provides a method for easily and rapidly quantifying the expression levels of the P450 molecular species under the same conditions, which is extremely useful especially in the development of new medicines.

The method of the present invention is also effective for investigating the mRNA expression of the P450 molecular species in an isolated tissue or a tissue obtained by biopsy. Thus, the method of the present invention is advantageous in that it can easily and rapidly process a variety of samples and achieve the desired detection using the total RNA extracted from a small amount of tissue.

There is a possibility that the change in the expression level of the P450 molecular species by enzyme induction or other process in the liver, kidney or the like correlates with the change of the expression level in the blood (nuclear cells in blood). However, since the expression level in blood is low, a large amount of blood is required for the measurement. The measurement method of the present invention, which uses any set of the above primer pairs and probes designed by the present inventors, is capable of easily and rapidly detecting and quantifying the P450 molecular species using a small amount of sample and common equipment. Therefore, the method of the present invention can detect and quantify the gene encoding the P450 molecular species in blood or a similar sample, thereby making it possible to estimate the expression levels of the molecular species.

### Probes and primers of the present invention

The probes and primers of the present invention each comprise an oligonucleotide hybridizable with a specific region of the CYP1A1 gene.

As used herein, the term "hybridizable" means being capable of hybridizing with the specific region under the following PCR (RT-PCR) conditions: 1 cycle of 48°C for 30 minutes, 1 cycle of 95°C for 10 minutes, and 50 cycles each consisting of 95°C for 15 seconds and 60°C for 1 minute.

Generally, the hybridizable oligonucleotide has a nucleotide sequence complementary to that of the specific region. However, it is known in this field that even if a primer or probe made of, for example, about 20 nucleotides, has about 1 or 2 mismatches with the template strand, the primer or probe hybridizes with the template strand and thus functions as a PCR primer or a detection probe. Accordingly, the primers and probes of the present invention include those comprising a nucleotide sequence with a small number of mismatches. However, the number of mismatches is preferably as small as possible.

The probes and primers of the present invention are required to have features that: they yield amplification products with a length of about 50 to about 400 bp; that the primers in each set is as close as possible to the probe; that the proportion of G (guanine) and C (cytosine) in each sequence is as close as possible to 50%; and that they do not contain four or more G (guanine) nucleotides in a row. Another feature required of the probes of the present invention is that they have a Tm of about 70°C. Further, the primers are required to have a Tm of about 60°C.

The number of nucleotides in the oligonucleotide for each primer or probe satisfying the above requirements is at least 15, usually 15 to 50, preferably 20 to 40. If the number of nucleotides in the primer or probe is much larger than the above range, the primer or probe is difficult to hybridize with a single-stranded DNA. On the other hand, if the number of nucleotides is too small, the hybridization specificity reduces.

Specific examples of nucleotide sequences for the primers and probes are as described above. The sequences shown in SEQ ID NO 1 (probes) and SEQ ID NOS: 36 to 37 (primers) are used.

Preferably, the oligonucleotides for use as the probes and primers of the present invention consist of a nucleotide sequence shown in these SEQ ID NOS. However, they are not limited to such oligonucleotides, and may have a small number of mismatches as long as they predominantly comprise one of these nucleotide sequences.

The mRNA sequences of P450 molecular species are known, and are registered in GenBank under the following accession numbers.
(1) The CYP1A1 gene: NM_000499

In this specification, the specific regions of the molecular species are indicated by the gene sequences registered under the above accession numbers.

The oligonucleotides for use as the probes and primers of the present invention can be easily synthesized in a routine manner, using an automatic synthesizer, such as a DNA synthesizer (Perkin-Elmer) or the like. The obtained oligonucleotides can be purified using a commercially available purification cartridge or the like, as desired.

### Real-time quantification and detection

The real-time P450 molecular species detection method by PCR using the primer pairs and probes of the present invention is described below in detail.

The real-time detection probes of the present invention comprise a reporter dye attached to one end, for example, the 5' end, and a quencher dye attached to the other end, for example, the 3' end. The reporter dye emits fluorescence, for example, upon irradiation with excitation light, and the quencher dye acts on the reporter dye to suppress the fluorescence emission when it is in close proximity to the reporter dye. Examples of reporter dyes include 6-carboxyfluorescein (FAM), tetrachloro-6-carboxyfluorescein (TET), 2,7-dimethoxy-4,5-dichloro-6-carboxyfluorescein (JOE), hexachloro-6-carboxyfluorescein (HEX), and the like. Examples of quencher dyes include 6-carboxytetramethylrhodamine (TAMRA) and the like.

A probe of the present invention can be prepared by attaching a reporter dye and a quencher dye to an oligonucleotide having the specific sequence. For example, a FAM molecule can be attached in the form of phosphoric acid ester to the phosphoric acid group at the 5' end of the probe, usually using several methylene chains as a linker. Further, a TAMRA molecule can be attached to the 3' end by an amide bond via the following structural unit.

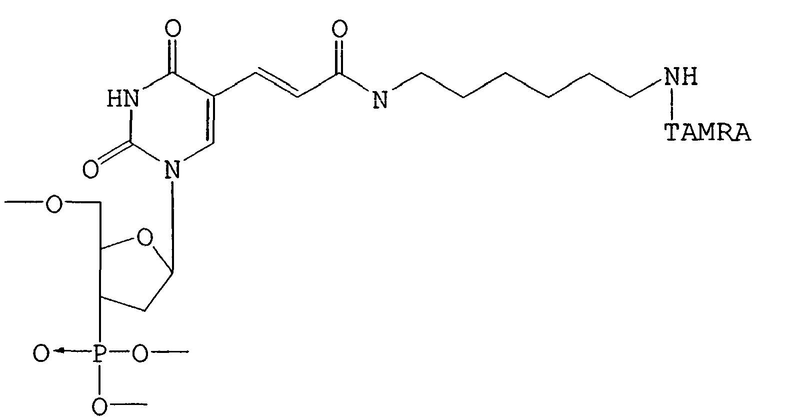

It is essential for the method of the present invention to use one or more of the primer pairs and probes of the present invention. Otherwise, the method can be conducted according to the known PCR (for example, Science, 230, 1350 (1985)) or RT-PCR (Genome Res., 6 (10), 986 (1996)), in particular, the real-time detection technique (for example, TaqMan PCR, ABI PRISMTM 7700 SEQUENCE DETECTION SYSTEM, Applied Biosystems, Ver1, June 1996).

The method of the present invention is especially useful for measuring the mRNA or cDNA of the target P450 molecular species, to determine the expression level of the P450 molecular species. In this case, a tissue expressing specific molecular species is cut out and the total RNA is extracted in a routine manner. Then, a cDNA complementary to the RNA is synthesized in a routine manner, followed by PCR using one or more of the primer pairs and probes of the present invention. Alternatively, after the extraction of total RNA in a routine manner, the RNA can be directly subjected to RT-PCR using one or more of the primers and probes of the present invention.

PCR and RT-PCR can be performed basically according to the known technique. The PCR or RT-PCR conditions can be selected according to the known technique and are similar to those employed therein. Specifically, the conditions shown in Examples given hereinafter can be preferably employed.

The detection can also be conducted basically according to the conventional technique, for example, by irradiating the PCR mixture with argon laser light and detecting the emitted fluorescence using a CCD camera.

The method of the present invention can easily and rapidly measure and detect the respective genes of P450 molecular species.

### Kit for detecting and quantifying the P450 molecular species

The present invention also provides a kit for performing the above method. The kit comprises the set of the above primer pairs and probes. The kit may further contain a known nucleic acid for use as a control, and a primer pair and probe for measuring the nucleic acid.

The method of the present invention can detect and quantify the P450 molecular species individually. Moreover, the method of the present invention can rapidly and accurately quantify the P450 molecular species. Accordingly, the application of the method of the present invention makes it possible to efficiently obtain fundamental data on the interaction and incompatibility of a new medicine with another medicine in humans.

### BEST MODE FOR CARRYING OUT THE INVENTION

Test Examples and Examples are given below to illustrate the present invention in further detail.

### Test Example 1 Preparation of calibration curves by the real-time detection technique

### (1) Test method

The real-time one-step RT-PCR technique employed in this test can inspect up to 96 samples at a time, by conducting up to 96 different reactions using 96 wells. Also, by this technique, RT and PCR can be performed in one tube.

The quantification is performed using two fluorescent dyes in close proximity, based on FRET produced when the wavelength regions of the fluorescence wavelength of one dye (reporter dye), and the excitation light wavelength of the other dye (quencher dye) overlap with each other. A probe (TaqMan probe), in which the two dyes causing FRET are attached to the ends, hybridizes with a cDNA derived from a particular P450 molecular species and amplified by PCR. The PCR extension reaction starts in this state, and the TaqMan probe is hydrolyzed by the 5'-3' endonuclease activity of TaqDNA polymerase, so that the reporter dye is released and the physical distance between the two dyes are increased. As a result, the fluorescence intensity of the reporter dye that has been suppressed by FRET is increased. Since the increase in the fluorescence intensity is proportional to the increase in amount of the PCR amplification product, the desired cDNA quantification can be achieved by measuring the increase in the fluorescence intensity after each PCR cycle.

In this test, FAM was attached as a reporter dye to the 5' end of the probe, and TAMRA was attached as a quencher dye to the 3' end. The attachment of these dyes and the preparation of the TaqMan probe were carried out by the techniques described in literature (Genome Res., 6 (10), 986 (1996)).

The oligonucleotides for use as the primers and probes were synthesized using an automatic DNA/RNA synthesizer (ABI), a dNTP substrate and prescribed reagents.

As sample RNAs, the following RNAs were isolated, purified and used in the form of total RNAs: CYP4B1 and CYP2F1 from the lung; CYP1B1 from the small intestine; CYP3A7 from a fetal liver pool; CYP4F8 from the prostate gland; CYP19 from the placenta; and CYP11B1, CYP11B2, and CYP17 from the adrenal gland. As sample RNAs of other molecular species, total RNAs purified from an adult liver pool was used. All these total RNAs were purchased from Clontech Laboratories, Inc.

The total RNAs were diluted with RNase-free water to 20 µg/mL, and then five-fold serially diluted with yeast tRNA (GIBCO) having a concentration of 50 µg/mL. Five microliters of each solution was used in the measurement.

RT-PCR was performed in a 50 µL/tube system, using TaqMan One-Step RT-PCR Master Mix Reagents Kit (PE Applied Biosystems) comprising a 300nM forward primer, a 900nM reverse primer, and a 200nM TaqMan probe, and ABI PRISM™7700 Sequence Detection System (PE Applied Biosystems).

The PCR conditions were as follows: 1 cycle of 48°C for 30 minutes, 1 cycle of 95°C for 10 minutes, and 50 cycles each consisting of 95°C for 15 seconds and 60°C for 1 minute. The fluorescence intensity was measured after each cycle.

Table 1 shows the target molecular species and primer pairs and probes used in the above test. Table 2 presents the test results (calibration curves).

**Table 1**

| No. | Molecular species | Primer pair | | Probe |
|---|---|---|---|---|
| | | Forward primer | Reverse primer | |
| (1)* | CYP1A1 | SEQ ID NO: 36 | SEQ ID NO: 37 | SEQ ID NO: 1 |
| (2) | CYP1A2 | SEQ ID NO: 38 | SEQ ID NO: 39 | SEQ ID NO: 2 |
| (3) | CYP1B1 | SEQ ID NO: 40 | SEQ ID NO: 41 | SEQ ID NO: 3 |
| (4) | CYP2A6/7 | SEQ ID NO: 42 | SEQ ID NO: 43 | SEQ ID NO: 4 |
| (5) | CYP2A6 | SEQ ID NO: 44 | SEQ ID NO: 45 | SEQ ID NO: 5 |
| (6) | CYP2A7 | SEQ ID NO: 46 | SEQ ID NO: 47 | SEQ ID NO: 6 |
| (7) | CYP2B6 | SEQ ID NO: 48 | SEQ ID NO: 49 | SEQ ID NO: 7 |
| (8) | CYP2C8 | SEQ ID NO: 50 | SEQ ID NO: 51 | SEQ ID NO: 8 |
| (9) | CYP2C9 | SEQ ID NO: 52 | SEQ ID NO: 53 | SEQ ID NO: 9 |
| (10) | CYP2C18 | SEQ ID NO: 54 | SEQ ID NO: 55 | SEQ ID NO: 10 |
| (11) | CYP2C19 | SEQ ID NO: 56 | SEQ ID NO: 57 | SEQ ID NO: 11 |
| (12) | CYP2D6 | SEQ ID NO: 58 | SEQ ID NO: 59 | SEQ ID NO: 12 |
| (13) | CYP2E1 | SEQ ID NO: 60 | SEQ ID NO: 61 | SEQ ID NO: 13 |
| (14) | CYP2F1 | SEQ ID NO: 62 | SEQ ID NO: 63 | SEQ ID NO: 14 |
| (15) | CYP2J2 | SEQ ID NO: 64 | SEQ ID NO: 65 | SEQ ID NO: 15 |
| (16) | CYP3A3/4 | SEQ ID NO 66 | SEQ ID NO: 67 | SEQ ID NO: 16 |
| (17) | CYP3A4 | SEQ ID NO: 68 | SEQ ID NO: 69 | SEQ ID NO: 17 |
| (18) | CYP3A5 | SEQ ID NO: 70 | SEQ ID NO: 71 | SEQ ID NO: 18 |
| (19) | CYP3A7 | SEQ ID NO: 72 | SEQ ID NO: 73 | SEQ ID NO: 19 |
| (20) | CYP4A11 | SEQ ID NO: 74 | SEQ ID NO: 75 | SEQ ID NO: 20 |
| (21) | CYP4B1 | SEQ ID NO: 76 | SEQ ID NO: 77 | SEQ ID NO: 21 |
| (22) | CYP4F2 | SEQ ID NO 78 | SEQ ID NO: 79 | SEQ ID NO: 22 |
| (23) | CYP4F3 | SEQ ID NO: 80 | SEQ ID NO: 81 | SEQ ID NO: 23 |
| (24) | CYP4F8 | SEQ ID NO: 82 | SEQ ID NO: 83 | SEQ ID NO: 24 |
| (25) | CYP11B1 | SEQ ID NO: 84 | SEQ ID NO: 85 | SEQ ID NO: 25 |
| (26) | CYP11B2 | SEQ ID NO: 86 | SEQ ID NO: 87 | SEQ ID NO: 26 |
| (27) | CYP17 | SEQ ID NO: 88 | SEQ ID NO: 89 | SEQ ID NO: 27 |
| (28) | CYP19 | SEQ ID NO: 90 | SEQ ID NO: 91 | SEQ ID NO: 28 |
| (29) | CYP27 | SEQ ID NO: 92 | SEQ ID NO: 93 | SEQ ID NO: 29 |
| (30) | CYP1B1 | SEQ ID NO: 94 | SEQ ID NO: 95 | SEQ ID NO: 30 |
| (31) | CYP2B6 | SEQ ID NO: 96 | SEQ ID NO: 97 | SEQ ID NO: 31 |
| (32) | CYP2C8 | SEQ ID NO: 98 | SEQ ID NO: 99 | SEQ ID NO: 32 |
| (33) | CYP2C9 | SEQ ID NO: 100 | SEQ ID NO: 101 | SEQ ID NO: 33 |
| (34) | CYP2C19 | SEQ ID NO: 102 | SEQ ID NO: 103 | SEQ ID NO: 34 |
| (35) | CYP4A11 | SEQ ID NO: 104 | SEQ ID NO: 105 | SEQ ID NO: 35 |

| | | | | |
|---|---|---|---|---|
| * target of the present invention | | | | |

**Table 2**

| No. | Calibration curve | | Coefficient of correlation (r) | Limit of quantification (pg total RNA) |
|---|---|---|---|---|
| | Slope | intercept | | |
| (1)* | -3.20 | 40.68 | 1.00 | 32 |
| (2) | -3.19 | 34.63 | 1.00 | 1.28 |
| (3) | -5.31 | 47.40 | 0.99 | 4000 |
| (4) | -3.60 | 33.90 | 0.99 | 32 |
| (5) | -3.07 | 32.93 | 1.00 | 6.4 |
| (6) | -3.12 | 37.32 | 1.00 | 1.28 |
| (7) | -3.43 | 36.31 | 0.99 | 160 |
| (8) | -3.52 | 35.62 | 0.99 | 32 |
| (9) | -3.51 | 37.23 | 1.00 | 800 |
| (10) | -3.18 | 33.95 | 0.99 | 160 |
| (11) | -3.41 | 35.97 | 1.00 | 6.4 |
| (12) | -3.17 | 37.70 | 0.99 | 160 |
| (13) | -3.19 | 31.21 | 1.00 | 1.28 |
| (14) | -3.54 | 44.49 | 0.99 | 800 |
| (15) | -3.44 | 39.41 | 1.00 | 6.4 |
| (16) | -3.15 | 35.11 | 1.00 | 1.28 |
| (17) | -3.36 | 36.38 | 1.00 | 1.28 |
| (18) | -3.30 | 41.48 | 1.00 | 160 |
| (19) | -3.60 | 38.53 | 1.00 | 32 |
| (20) | -4.20 | 38.79 | 0.96 | 4000 |
| (21) | -3.22 | 36.23 | 1.00 | 160 |
| (22) | -2.95 | 35.24 | 1.00 | 1.28 |
| (23) | -3.49 | 37.21 | 0.99 | 6.4 |
| (24) | -3.41 | 37.97 | 1.00 | 6.4 |
| (25) | -3.26 | 36.35 | 1.00 | 1.28 |
| (26) | -3.02 | 36.02 | 1.00 | 32 |
| (27) | -3.18 | 30.32 | 1.00 | 1.28 |
| (28) | -3.21 | 31.07 | 1.00 | 1.28 |
| (29) | -3.39 | 37.65 | 1.00 | 6.4 |
| (30) | -3.34 | 43.42 | 1.00 | 160 |
| (31) | -3.33 | 36.84 | 1.00 | 1.28 |
| (32) | -3.29 | 34.53 | 1.00 | 1.28 |
| (33) | -3.29 | 34.43 | 1.00 | 1.28 |
| (34) | -3.52 | 41.10 | 1.00 | 6.4 |
| (35) | -3.28 | 35.08 | 1.00 | 1.28 |

| | | | | |
|---|---|---|---|---|
| * target of the present invention | | | | |

In Table 2, the calibration curves prepared from RNA solutions that were five-fold serially diluted from 100000 pg total RNA/50 µL reaction mixture show that: CYP3A4 is quantifiable at a concentration as low as 1.28 pg total RNA/50 µL reaction mixture; and that the coefficients of correlation (r) of the calibration curves of other molecular species were 0.99 or higher when the limit of quantification was 1.28 pg to 4000 pg total RNA. However, the coefficient of correction of the calibration curve of CYP4A11 was 0.96.

### Test Example 2 Confirmation of P450 molecular species specificity of the primer-probe sets designed

### (1) Test method

This test was conducted as follows, to demonstrate that the method of the present invention can specifically distinguish P450 molecular species. The expression of P450 molecular species in tissues of the adrenal gland, liver, fetal liver, small intestine, kidney, lung, brain, prostate gland, testis, uterus and placenta was tested by the method of the present invention, in the same manner as in Test Example 1. The obtained results are compared with the results reported in literature.

GAPDH (glyceraldehyde-3-phosphate dehydrogenase) was used as the internal standard, and the test was performed in triplicate.

The total RNA pools of the tissues were purchased from Clontech Laboratories, Inc.

The total RNA concentration was 20000 pg total RNA/50 µL reaction mixture, for the tissues used for preparation of the calibration curves, and 100000 pg total RNA/50 µL reaction mixture, for other tissues.

Tables 3 and 4 present the results. The tables also show the characteristics (pooled number, age, sex, race, and cause of death) of the derivations of the total RNAs. In the tables, "ND" indicates less than the limit of quantification.

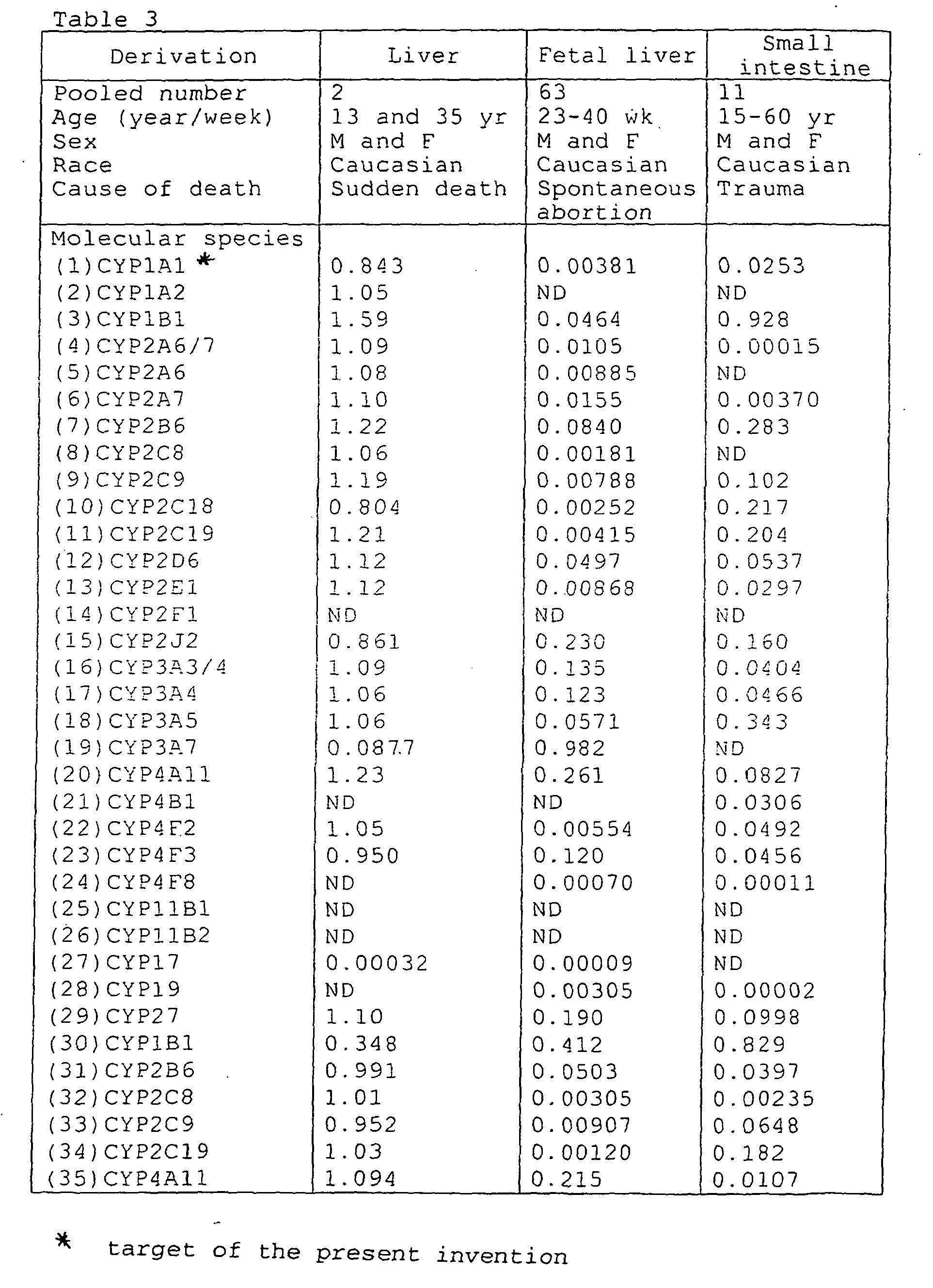

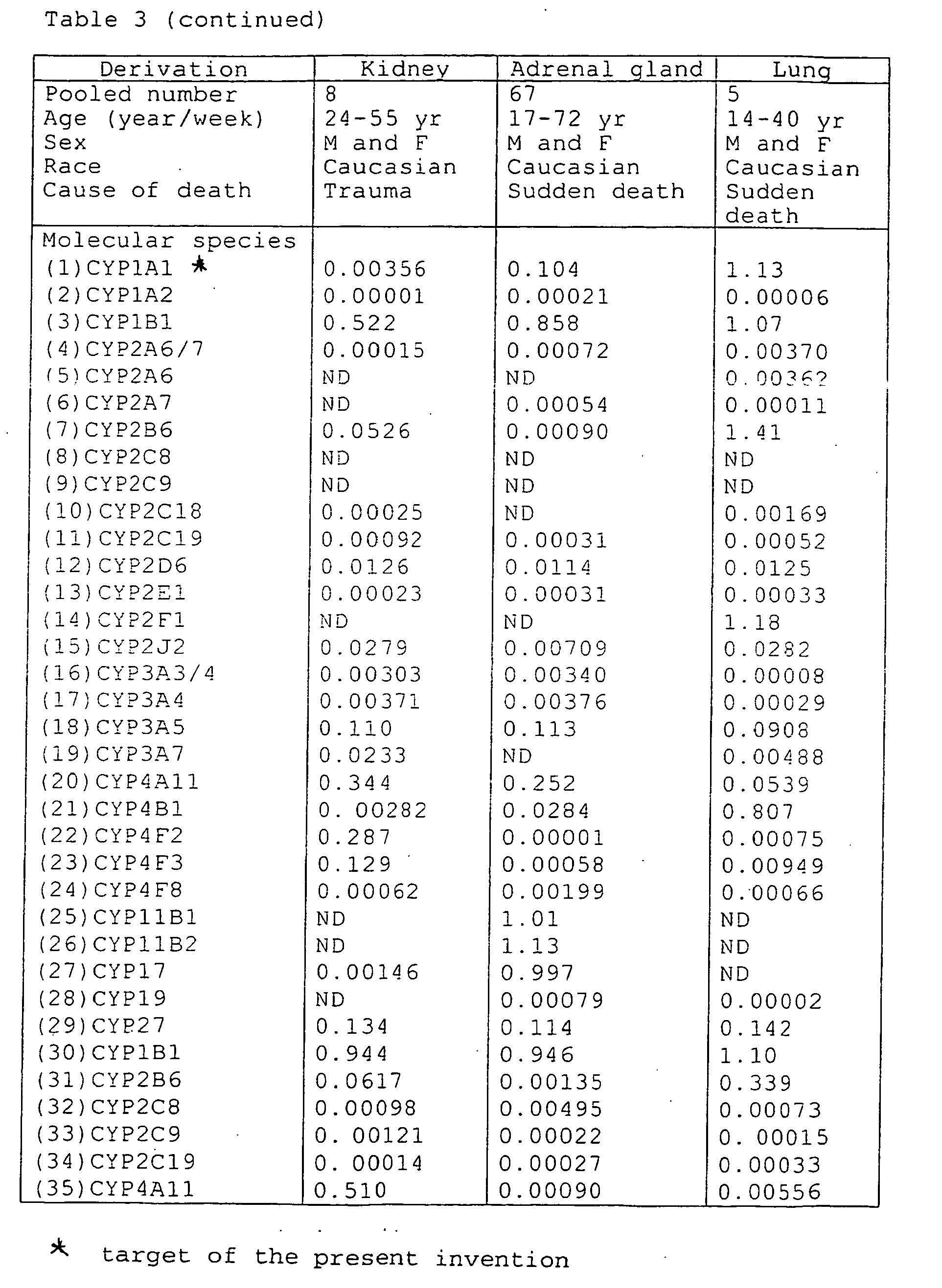

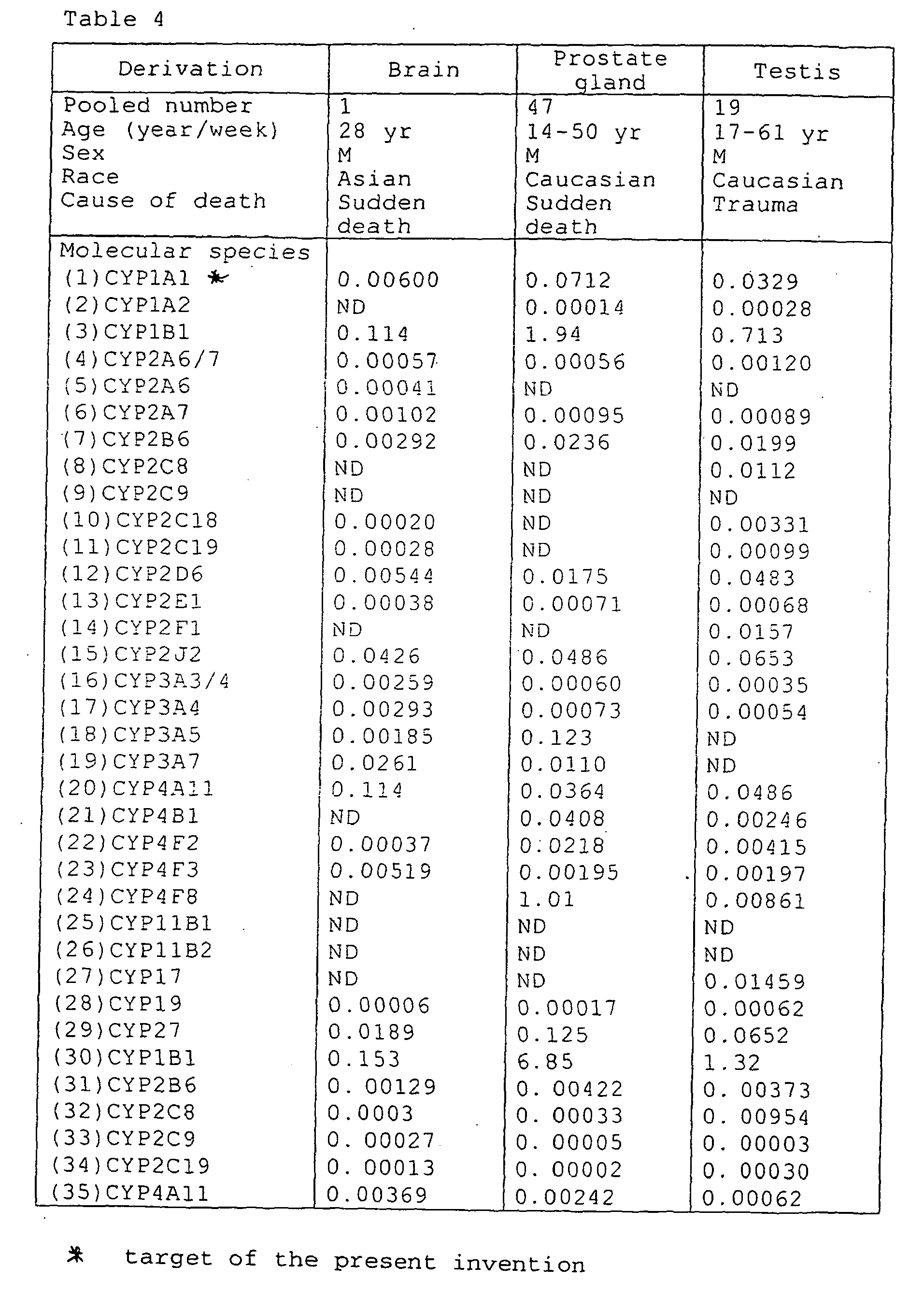

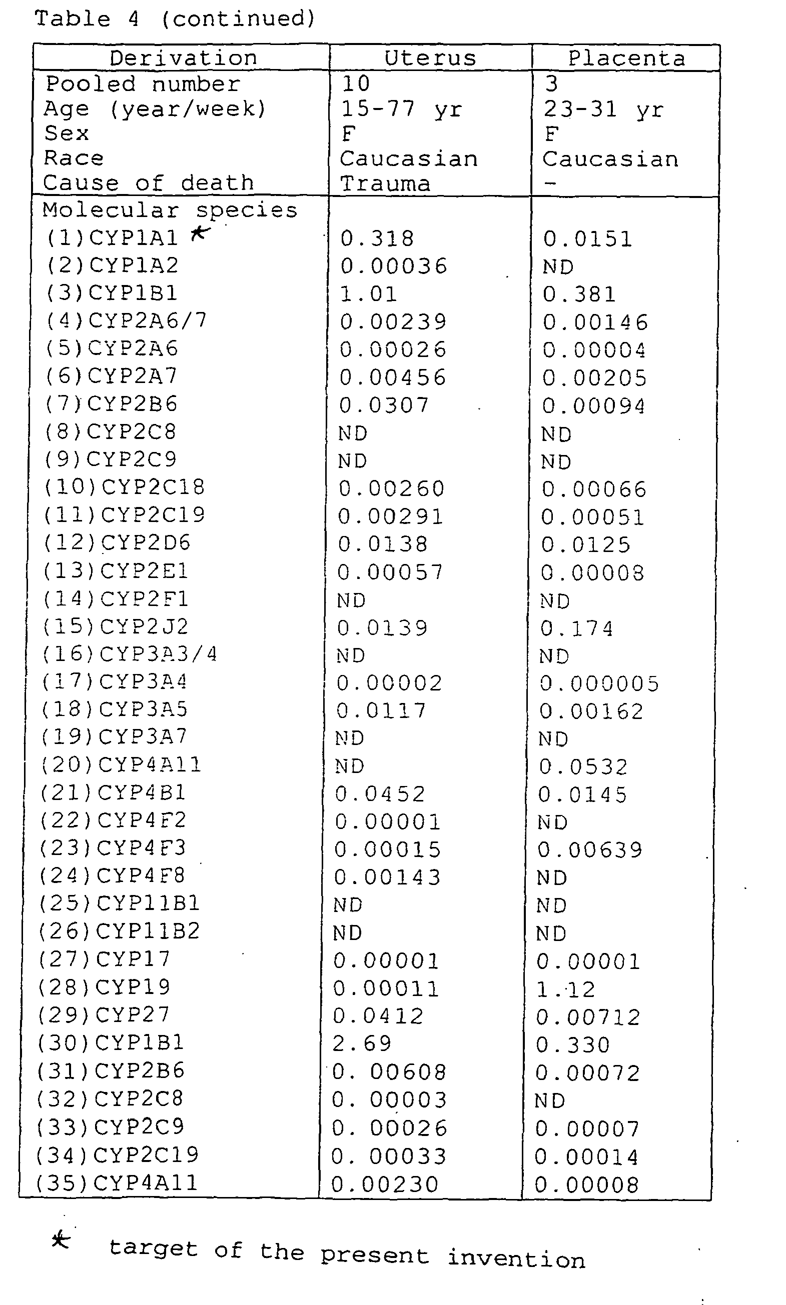

The results shown in the tables were compared with those obtained by the prior art techniques reported in the following Documents 1 to 5, and found to substantially correlate therewith. Further, molecular species that cannot be detected by the prior art techniques can be detected by the method of the present invention. The results achieved by the method of the present invention exhibit tissue-specific expression patterns that do not overlap with one another. Thus, it was demonstrated that the method of the present invention is capable of quantifying P450 molecular species individually.

| | |
|---|---|
| Document 1: | Drug Metabolism and Disposition 27, 804-809 (1999) |
| Document 2: | Exp. Toxic. Pathol., 51, 412-417 (1999) |
| Document 3: | Biochemical Pharmacology, 52, 379-383 (1996) |
| Document 4: | Biochemical Pharmacology, 57, 1407-1413 (1999) |
| Document 5: | Pharmacology & Therapeutics, 84, 429-445 (1999) |

### INDUSTRIAL APPLICABILITY

The present invention provides a method for easily and rapidly detecting and quantifying human P450 molecular species individually in real time under the same PCR reaction conditions, and probes and primers for use in the method. The method of the present invention is useful in the clinical and medical fields.

## Claims

1. A kit for detecting and quantifying the CYP1A1 gene of the human cytochrom P450 molecular species, comprising a set of a primer pair of a forward primer comprising an oligonucleotide which hybridizes with the 589-610 region of the CYP1A1 gene and a reverse primer comprising an oligonucleotide which hybridizes with the 685-664 region of the CYP1A1 gene, and a probe comprising an oligonucleotide which hybridizes with the 616-641 region of the CYP1A1 gene.

2. A kit according to claim 1, wherein the probe further comprises a reporter dye and a quencher dye attached thereto.

3. A kit according to claim 2, which comprises a set of a primer pair comprising the sequences shown in SEQ ID Nos: 36 and 37 and a probe comprising the sequence shown in SEQ ID No: 1.

4. A kit according to claim 2, which comprises a set of a primer pair consisting of the sequences shown in SEQ ID Nos: 36 and 37 and a probe consisting of the sequence shown in SEQ ID No: 1.

5. A method of detecting and quantifying the CYP1A1 gene of the human cytochrome P450 molecular species, the method comprising the steps of:
(a) preparing a sample containing a human cytochrome P450 gene or genes,
(b) subjecting the sample to a polymerase chain reaction using a kit according to any one of claims 1 to 4 to amplify the CYP1A1 gene of the human cytochrom P450 molecular species in the sample; and
(c) detecting and measuring the probe or probes hydrolyzed during the polymerase chain reaction.

6. A method according to claim 5, wherein the hydrolyzed probe or probes are detected and quantified by detecting and measuring the fluorescence produced by irradiation with excitation light.

## Patentansprüche

1. Kit zum Nachweis und zur Quantifizierung des CYP1A1-Gens der menschlichen Cytochrom P450 molekularen Spezies, umfassend einen Satz eines Primerpaars eines Vorwärts-Primers, umfassend ein Oligonukleotid, das mit der 589-610 Region des CYP1A1-Gens hybridisiert, und einen Rückwärts-Primer, umfassend ein Oligonukleotid, das mit der 685-664 Region des CYP1A1-Gens hybridisiert, und eine Sonde, umfassend ein Oligonukleotid, das mit der 616-641 Region des CYP1A1-Gens hybridisiert.

2. Kit gemäß Anspruch 1, wobei die Sonde weiterhin einen Reporterfarbstoff und einen Quencherfarbstoff umfaßt, daran angebunden.

3. Kit gemäß Anspruch 2, das einen Satz einen Primerpaars umfaßt, umfassend die in SEQ ID NO: 36 und 37 dargestellten Sequenzen, und eine Sonde, umfassend die in SEQ ID NO: 1 dargestellte Sequenz.

4. Kit gemäß Anspruch 2, das einen Satz eines Primerpaars umfaßt, bestehend aus den Sequenzen wie dargestellt in SEQ ID NO: 36 und 37 und eine Sonde, bestehend aus der in SEQ ID NO: 1 dargestellten Sequenz.

5. Verfahren zum Nachweis und der Quantifizierung des CYP1A1-Gens der menschlichen Cytochrom P450 molekularen Spezies, wobei das Verfahren die folgenden Schritte umfaßt:
(a) Herstellung einer Probe, enthaltend ein menschliches Cytochrom P450-Gen oder -Gene;
(b) Unterwerfen der Probe einer Polymerasekettenreaktion unter Verwendung eines Kits gemäß einem der Ansprüche 1 bis 4, um das CYP1A1-Gen der menschlichen Cytochrom P450 molekularen Spezies in der Probe zu amplifizieren und
(c) Nachweis und Messung der Sonde oder Sonden, die während der Polymerasekettenreaktion hydrolysiert sind.

6. Verfahren gemäß Anspruch 5, wobei die hydrolysierte Sonde oder Sonden durch Nachweis und Messung der Fluoreszenz, erzeugt durch Bestrahlung mit anregendem Licht, nachgewiesen und quantifiziert werden.

## Revendications

1. Trousse pour détecter et quantifier le gène CYP1A1 de l'espèce moléculaire cytochrome P450 humain, comprenant un ensemble de paire d'amorces d'une amorce sens comprenant un oligonucléotide qui s'hybride à la région 589-610 du gène CYP1A1 et une amorce antisens comprenant un oligonucléotide qui s'hybride à la région 685-664 du gène CYP1A1, et une sonde comprenant un oligonucléotide qui s'hybride à la région 616-641 du gène CYP1A1.

2. Trousse selon la revendication 1, dans laquelle la sonde comprend en outre un colorant rapporteur et un colorant désactivateur fixé à elle.

3. Trousse selon la revendication 2, qui comprend un ensemble d'une paire d'amorces comprenant les séquences présentées dans SEQ ID N° 36 et 37 et une sonde comprenant la séquence présentée dans SEQ ID N° 1.

4. Trousse selon la revendication 2, qui comprend un ensemble d'une paire d'amorces consistant en les séquences présentées dans SEQ ID N° 36 et 37 et une sonde consistant en la séquence présentée en SEQ ID N° 1.

5. Procédé de détection et de quantification du gène CYP1A1 de l'espèce moléculaire cytochrome P450 humain, le procédé comprenant les étapes suivantes :
(a) préparer un échantillon contenant un ou plusieurs gènes du cytochrome P450 humain,
(b) soumettre l'échantillon à une réaction de polymérisation en chaîne en utilisant une trousse selon l'une quelconque des revendications 1 à 4 pour amplifier le gène CYP1A1 de l'espèce moléculaire cytochrome P450 humain dans l'échantillon ; et
(c) détecter et mesurer la sonde ou les sondes hydrolysées pendant la réaction de polymérisation en chaîne.

6. Procédé selon la revendication 5, dans lequel la ou les sondes hydrolysées sont détectées et quantifiées en détectant et en mesurant la fluorescence produite par l'irradiation par une lumière d'excitation.
